# EUROPEAN PATENT SPECIFICATION

(11) **EP 4 117 520 B1**
(45) Date of publication and mention of the grant of the patent: **09.07.2025**
(21) Application number: 21720596.2
(22) Date of filing: 11.03.2021
(51) Int. Cl.: G01N 33/487, A61B 5/1468, G16H 50/30, G16H 20/60, G16H 40/63, G16H 15/00, G16H 20/17, G16H 40/67, A61B 5/00, A61B 5/145

(54) **GRAPHICAL USER INTERFACES FOR ANALYTE MONITORING SYSTEMS**
GRAFISCHE BENUTZEROBERFLÄCHEN FÜR ANALYTÜBERWACHUNGSSYSTEME
INTERFACES UTILISATEUR GRAPHIQUES POUR LES SYSTÈMES DE SURVEILLANCE DES ANALYTES

(30) Priority: 11.03.2020 US 202062988326 P
(43) Date of publication of application: 18.01.2023
(73) Proprietor: Abbott Diabetes Care Inc., Alameda, California 94502 (US)
(72) Inventor: KUMAR, Panganamala Ashwin, Oakland, California 94618 (US); WOO, Jennifer, Oakland, California 94610 (US); ROSSI, Stephen A., Clayton, California 94517 (US); JANGAM, Sujit, San Mateo, California 94403 (US); COVINGTON, Kendall, Oakland, California 94609 (US); LANG, Jordan Wing-Haye, San Jose, California 95133 (US)
(74) Representative: Mathys & Squire
(86) International application number: PCT/US2021/021943
(87) International publication number: WO 2021/183784

(56) References cited:
- WO-A1-2011/026053
- US-A1- 2016 270 740
- US-A1- 2019 183 393
- US-A1- 2021 030 323
- US-B2- 10 772 503

## Description

### FIELD

The subject matter described herein relates generally to graphical user interfaces for analyte monitoring systems, as well as methods and devices relating thereto.

### BACKGROUND

The detection and/or monitoring of analyte levels, such as glucose, ketones, lactate, oxygen, hemoglobin A1C, or the like, can be vitally important to the health of an individual having diabetes. Patients suffering from diabetes mellitus can experience complications including loss of consciousness, cardiovascular disease, retinopathy, neuropathy, and nephropathy. Diabetics are generally required to monitor their glucose levels to ensure that they are being maintained within a clinically safe range, and may also use this information to determine if and/or when insulin is needed to reduce glucose levels in their bodies, or when additional glucose is needed to raise the level of glucose in their bodies.

Growing clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

To increase patient adherence to a plan of frequent glucose monitoring, in vivo analyte monitoring systems can be utilized, in which a sensor control device may be worn on the body of an individual who requires analyte monitoring. To increase comfort and convenience for the individual, the sensor control device may have a small form-factor and can be applied by the individual with a sensor applicator. The application process includes inserting at least a portion of a sensor that senses a user's analyte level in a bodily fluid located in a layer of the human body, using an applicator or insertion mechanism, such that the sensor comes into contact with a bodily fluid. The sensor control device may also be configured to transmit analyte data to another device, from which the individual or her health care provider ("HCP") can review the data and make therapy decisions.

Despite their advantages, however, some people are reluctant to use analyte monitoring systems for various reasons, including the complexity and volume of data presented, a learning curve associated with the software and user interfaces for analyte monitoring systems, and an overall paucity of actionable information presented.

Thus, needs exist for graphical user interfaces for analyte monitoring systems, as well as methods and devices relating thereto, that are robust, user-friendly, and provides for timely and actionable responses.

US2019/183393A1 describes analyte monitoring systems, devices, and methods associated with analyte monitoring devices, and devices incorporating the same. Various graphical user interfaces (GUI) and navigation flows are provided for performing various features, activities, functions, etc., associated with the analyte monitoring device or system. Intuitive navigation is provided to enhance the interpretation of analyte measurements.

WO2011/026053A1 describes an analyte monitoring device having a user interface with a display and a plurality of actuators. The display is configured to render a plurality of display screens, including a home screen and an alert screen. The home screen is divided into a plurality of simultaneously displayed panels, with a first panel displays a rate of change of continuously monitored analyte levels in interstitial fluid, a second panel simultaneously displays a current analyte level and an analyte trend indicator, and a third panel displays status information of a plurality of components of the device. When an alarm condition is detected, the display renders the alert screen in place of the home screen, the alert screen displaying information corresponding to the detected alarm condition. Furthermore, the actuators are configured to affect further output of the analyte monitoring device corresponding to the detected condition.

US2016/270740A1 describes a computing device that receives analyte data produced by an analyte monitoring sensor over a communications link from at least one first device. Health data, comprising at least part of the analyte data, may be communicated over a communications link to at least one second device in response to a request. The first device may be positioned over the analyte monitoring sensor using signal strength and location information. External analyte data may be employed to calibrate the analyte monitoring sensor.

### SUMMARY

The invention is defined by the appended claims. Provided herein are example embodiments of graphical user interfaces ("GUIs") for in vivo analyte monitoring systems. According to some embodiments, a Time-in-Ranges ("TIR") GUI of an analyte monitoring system is provided, wherein the TIR GUI comprises a plurality of bars or bar portions, wherein each bar or bar portion indicates an amount of time that a user's analyte level is within a predefined analyte range correlating with the bar or bar portion. In some embodiments, for example, the amount of time can be expressed as a percentage of total time. According to another embodiment, an Analyte Level/Trend Alert GUI of an analyte monitoring system is provided, wherein the Analyte Level/Trend Alert GUI comprises a visual notification (e.g., alert, alarm, pop-up window, banner notification, etc.), wherein the visual notification includes an alarm condition, an analyte level measurement associated with the alarm condition, and a trend indicator associated with the alarm condition. In some embodiments, for example, the trend indicator comprises a directional trend arrow.

The embodiments provided herein are improved GUIs or GUI features for analyte monitoring systems that are highly intuitive, user-friendly, and provide for rapid access to physiological information of a user. More specifically, these embodiments allow a user to easily navigate through and between different user interfaces that can quickly indicate to the user various physiological conditions and/or actionable responses, without requiring the user (or an HCP) to go through the arduous task of examining large volumes of analyte data. Other improvements and advantages are provided as well. The various configurations of these devices are described in detail by way of the embodiments which are only examples.

Other systems, devices, methods, features and advantages of the subject matter described herein will be or will become apparent to one with skill in the art upon examination of the following figures and detailed description. It is intended that all such additional systems, devices, methods, features, and advantages be included within this description, be within the scope of the subject matter described herein, and be protected by the accompanying claims. In no way should the features of the example embodiments be construed as limiting the appended claims, absent express recitation of those features in the claims.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is a system overview of a sensor applicator, reader device, monitoring system, network, and remote system.
FIG. 2A is a block diagram depicting an example embodiment of a reader device.
FIGS. 2B and 2C are block diagrams depicting example embodiments of sensor control devices.
FIGS. 3A to 3F are example embodiments of GUIs comprising time-in-ranges interfaces.
FIGS. 4A to 4O are example embodiments of GUIs comprising analyte level and trend alert interfaces.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include GUIs for analyte monitoring systems, and methods and devices relating thereto. Accordingly, many embodiments include in vivo analyte sensors structurally configured so that at least a portion of the sensor is, or can be, positioned in the body of a user to obtain information about at least one analyte of the body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices, reader devices, local computer systems, and trusted computer systems are disclosed, and these devices and systems can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps.

As previously described, a number of embodiments described herein provide for improved GUIs for analyte monitoring systems, wherein the GUIs are highly intuitive, user-friendly, and provide for rapid access to physiological information of a user. According to some embodiments, a Time-in-Ranges GUI of an analyte monitoring system is provided, wherein the Time-in-Ranges GUI comprises a plurality of bars or bar portions, wherein each bar or bar portion indicates an amount of time that a user's analyte level is within a predefined analyte range correlating with the bar or bar portion. According to another embodiment, an Analyte Level/Trend Alert GUI of an analyte monitoring system is provided, wherein the Analyte Level/Trend Alert GUI comprises a visual notification (e.g., alert, alarm, pop-up window, banner notification, etc.), and wherein the visual notification includes an alarm condition, an analyte level measurement associated with the alarm condition, and a trend indicator associated with the alarm condition. In sum, these embodiments provide for a robust, user-friendly interfaces that can increase user engagement with the analyte monitoring system and provide for timely and actionable responses by the user, to name a few advantages.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

### Example Embodiment of In Vivo Analyte Monitoring System

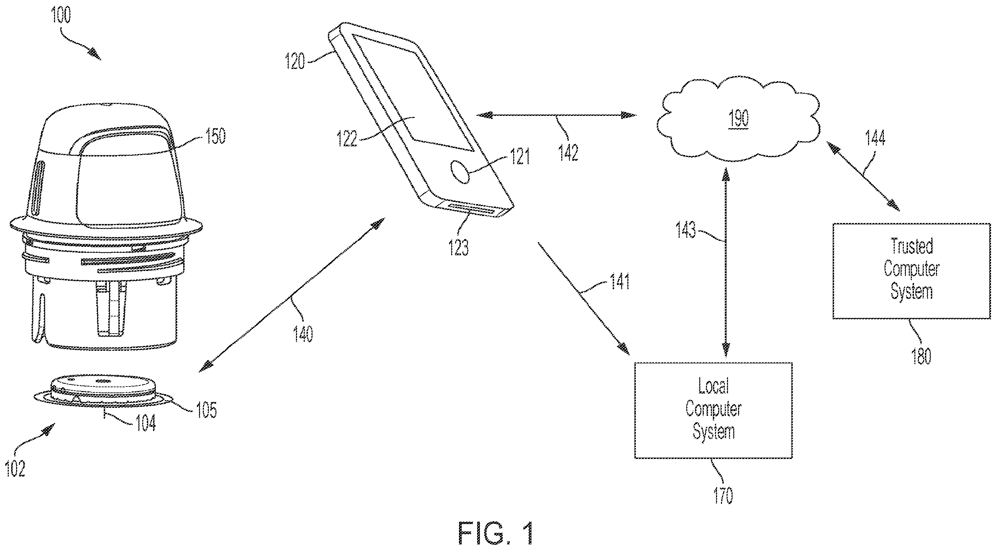

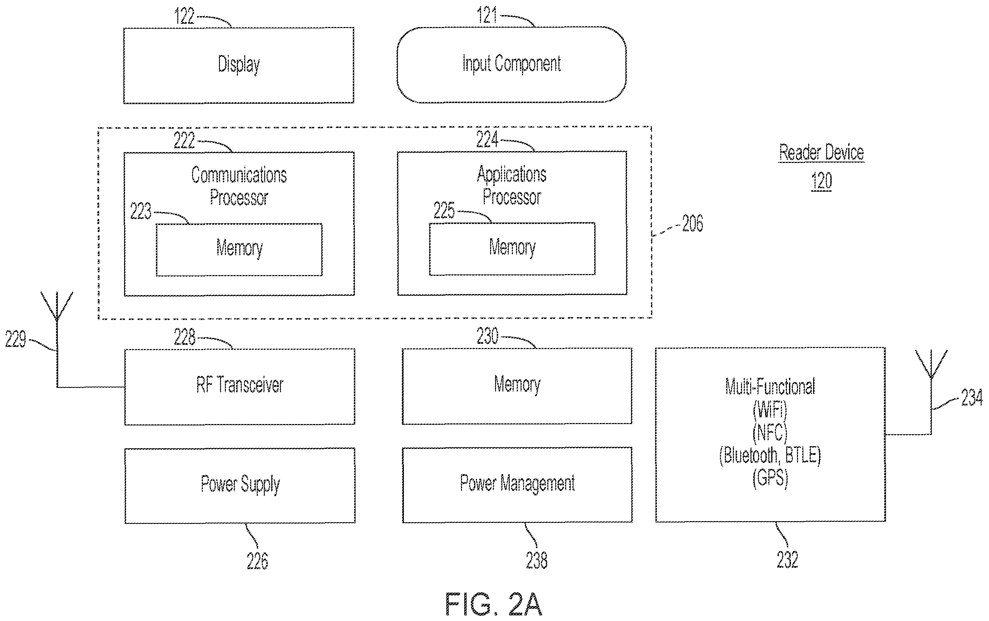

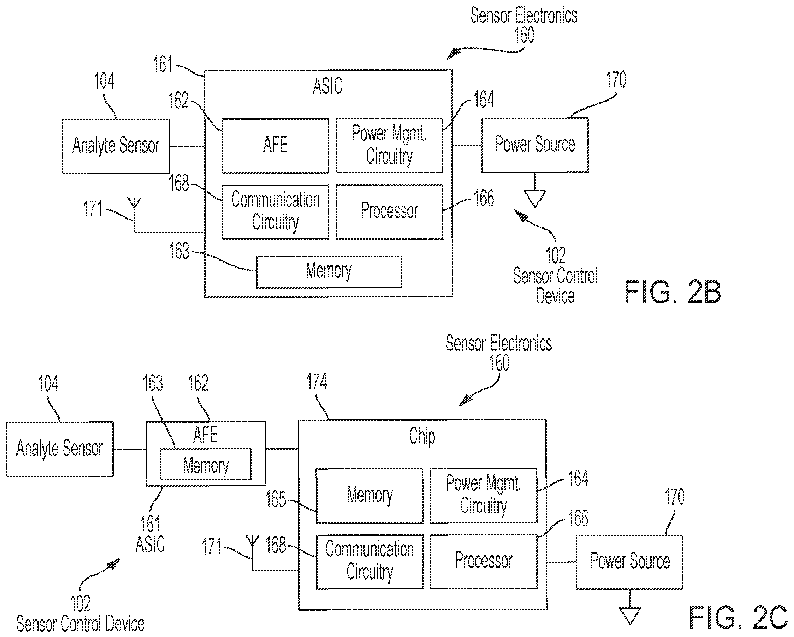

FIG. 1 is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a user's skin where a sensor 104 is maintained in position for a period of time by an adhesive patch 105. Sensor control device 102 is further described in FIGS. 2B and 2C, and can communicate with reader device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can view and use applications installed in memory on reader device 120 using screen 122 (which, in many embodiments, can comprise a touchscreen), and input 121. A device battery of reader device 120 can be recharged using power port 123. While only one reader device 120 is shown, sensor control device 102 can communicate with multiple reader devices 120. Each of the reader devices 120 can communicate and share data with one another. More details about reader device 120 is set forth with respect to FIG. 2A below. Reader device 120 can communicate with local computer system 170 via a communication path 141 using a wired or wireless communication protocol. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by a wired or wireless communication protocol as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

### Example Embodiment of Reader Device

FIG. 2A is a block diagram depicting an example embodiment of a reader device 120, which, in some embodiments, can comprise a smartphone. Here, reader device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further, reader device 120 can also include a multi-functional transceiver 232 which can communicate over Wi-Fi, NFC, Bluetooth, BTLE, and GPS with an antenna 234. As understood by one of skill in the art, these components are electrically and communicatively coupled in a manner to make a functional device.

### Example Embodiments of Sensor Control Devices

FIGS. 2B and 2C are block diagrams depicting example embodiments of sensor control devices 102 having analyte sensors 104 and sensor electronics 160 (including analyte monitoring circuitry) that can have the majority of the processing capability for rendering end-result data suitable for display to the user. In FIG. 2B, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or non-volatile memory. In this embodiment, ASIC 161 is coupled with power source 170, which can be a coin cell battery, or the like. AFE 162 interfaces with in vivo analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to reader device 120 (not shown), for example, where minimal further processing is needed by the resident software application to display the data.

FIG. 2C is similar to FIG. 2B but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

### Example Embodiments of User Interfaces for Analyte Monitoring Systems

Described herein are example embodiments of GUIs for analyte monitoring systems. As an initial matter, it will be understood by those of skill in the art that the GUIs described herein comprise instructions stored in a memory of reader device 120, local computer system 170, trusted computer system 180, and/or any other device or system that is part of, or in communication with, analyte monitoring system 100. These instructions, when executed by one or more processors of the reader device 120, local computer system 170, trusted computer system 180, or other device or system of analyte monitoring system 100, cause the one or more processors to perform the method steps and/or output the GUIs described herein. Those of skill in the art will further recognize that the GUIs described herein can be stored as instructions in the memory of a single centralized device or, in the alternative, can be distributed across multiple discrete devices in geographically dispersed locations.

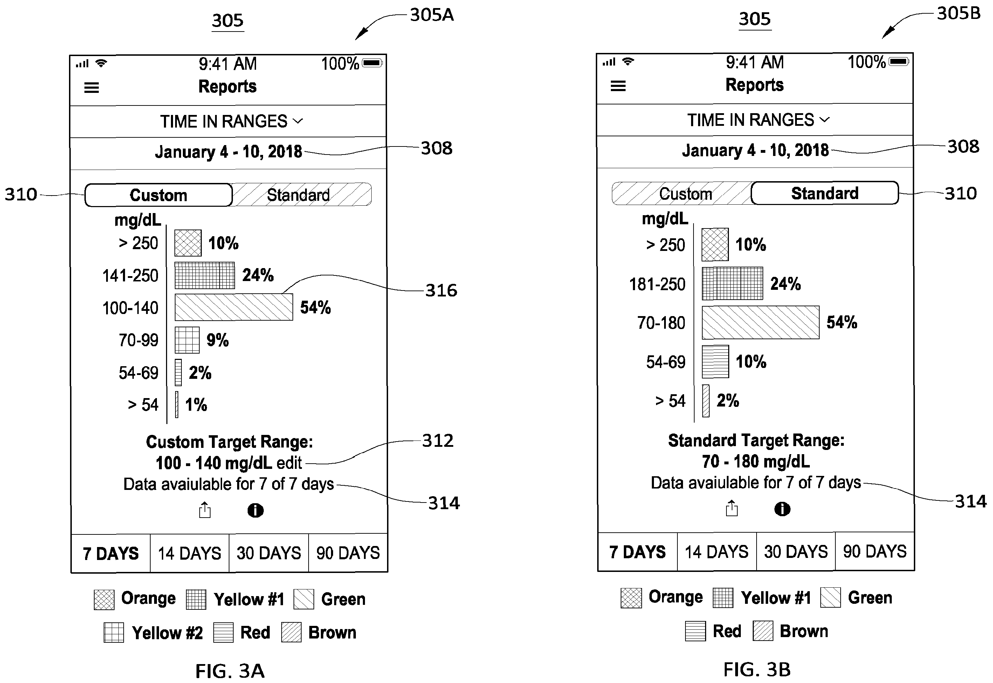

FIGS. 3A to 3F depict example embodiments of GUIs for analyte monitoring systems. In particular, FIGS. 3A to 3F depict Time-in-Ranges (also referred to as Time-in-Range and/or Time-in-Target) GUIs, each of which comprise a plurality of bars or bar portions, wherein each bar or bar portion indicates an amount of time that a user's analyte level is within a predefined analyte range correlating with the bar or bar portion. In some embodiments, for example, the amount of time can be expressed as a percentage of a predefined amount of time.

Turning to FIGS. 3A and 3B, an example embodiment of a Time-in-Ranges GUI 305 is shown, wherein Time-in-Ranges GUI 305 comprises a "Custom" Time-in-Ranges view 305A and a "Standard" Time-in-Ranges view 305B, with a toggle, switch, or slidable element 310 that allows the user to select between the two views. According to one aspect of the embodiments, Time-in-Ranges views 305A, 305B can each comprise multiple bars, wherein each bar indicates an amount of time that a user's analyte level is within a predefined analyte range correlating with the bar. In some embodiments, Time-in-Ranges views 305A, 305B further comprise a date range indicator 308, showing relevant dates associated with the displayed analyte data, and a data availability indicator 314, showing the period(s) of time in which analyte data is available for the displayed analyte data (e.g., "Data available for 7 of 7 days").

Referring to FIG. 3A, "Custom" Time-in-Ranges view 305A includes six bars comprising (from top to bottom): a first bar indicating that the user's glucose range is above 250 mg/dL for 10% of a predefined amount of time, a second bar indicating that the user's glucose range is between 141 and 250 mg/dL for 24% of the predefined amount of time, a third bar 316 indicating that the user's glucose range is between 100 and 140 mg/dL for 54% of the predefined amount of time, a fourth bar indicating that the user's glucose range is between 70 and 99 mg/dL for 9% of the predefined amount of time, a fifth bar indicating that the user's glucose range is between 54 and 69 mg/dL for 2% of the predefined amount of time, and a sixth bar indicating that the user's glucose range is less than 54 mg/dL for 1% of the predefined amount of time. Those of skill in the art will recognize that the glucose ranges and percentages of time associated with each bar can vary depending on the ranges defined by the user and the available analyte data of the user. Furthermore, although FIGS. 3A and 3B show a predefined amount of time 314 equal to seven days, those of skill in the art will appreciate that other predefined amounts of time can be utilized (e.g., one day, three days, fourteen days, thirty days, ninety days, etc.), and are fully within the scope of the present disclosure.

According to another aspect of the embodiments, "Custom" Time-in-Ranges view 305A also includes a user-definable custom target range 312 that includes an actionable "edit" link that allows a user to define and/or change the custom target range. As shown in "Custom" Time-in-Ranges view 305A, the custom target range 312 has been defined as a glucose range between 100 and 140 mg/dL and corresponds with third bar 316 of the plurality of bars.

Referring to FIG. 3B, "Standard" Time-in-Ranges view 305B includes five bars comprising (from top to bottom): a first bar indicating that the user's glucose range is above 250 mg/dL for 10% of a predefined amount of time, a second bar indicating that the user's glucose range is between 181 and 250 mg/dL for 24% of the predefined amount of time, a third bar indicating that the user's glucose range is between 70 and 180 mg/dL for 54% of the predefined amount of time, a fourth bar indicating that the user's glucose range is between 54 and 69 mg/dL for 10% of the predefined amount of time, and a fifth bar indicating that the user's glucose range is less than 54 mg/dL for 2% of the predefined amount of time. As with the "Custom" Time-in-Ranges view 305A, those of skill in the art will recognize that the percentages of time associated with each bar can vary depending on the available analyte data of the user. Unlike the "Custom" Time-in-Ranges view 305A, however, the glucose ranges shown in "Standard" view 305B cannot be adjusted by the user.

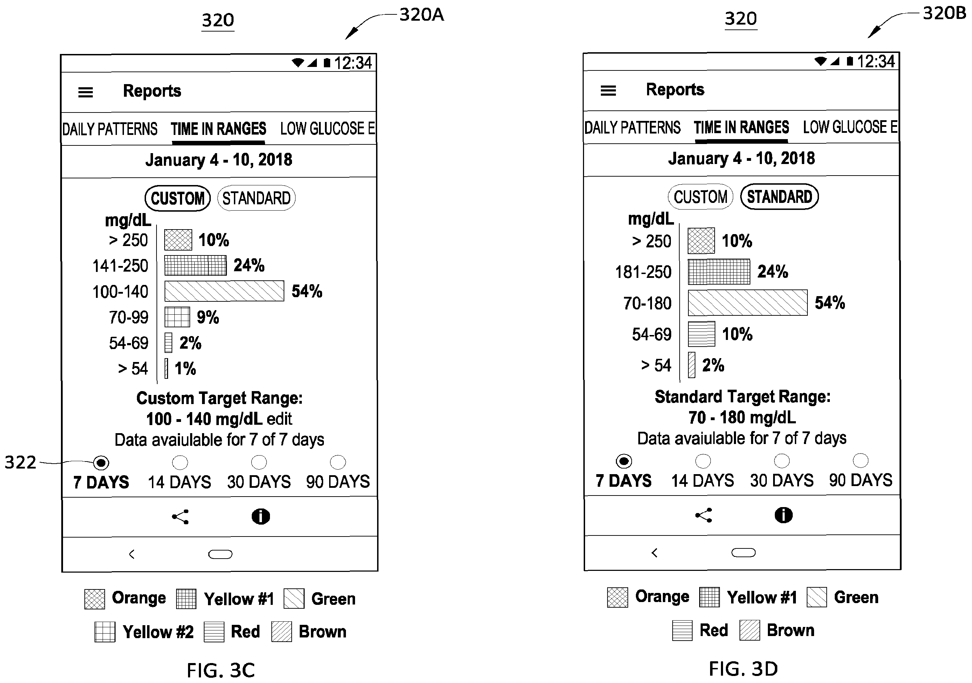

FIGS. 3C and 3D depict another example embodiment of Time-in-Ranges GUI 320 with multiple views, 320A and 320B, which are analogous to the views shown in FIGS. 3A and 3B, respectively. According to some embodiments, Time-in-Ranges GUI 320 can further include one or more selectable icons 322 (e.g., radio button, check box, slider, switch, etc.) that allow a user to select a predefined amount of time over which the user's analyte data will be shown in the Time-in-Range GUI 320. For example, as shown in FIGS. 3C and 3D, selectable icons 322 can be used to select a predefined amount of time of seven days, fourteen days, thirty days, or ninety days. Those of skill in the art will appreciate that other predefined amounts of time can be utilized and are fully within the scope of the present disclosure.

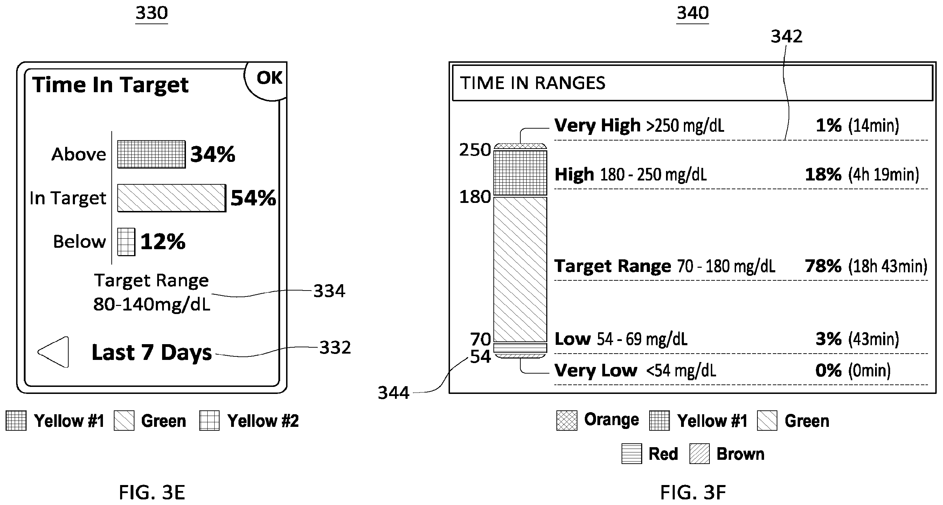

FIG. 3E depicts an example embodiment of a Time-in-Target GUI 330, which can be visually output to a display of a reader device (e.g., a dedicated reader device, a meter device, etc.). According to one aspect of the embodiments, Time-in-Target GUI 330 includes three bars comprising (from top to bottom): a first bar indicating that the user's glucose range is above a predefined target range for 34% of a predefined amount of time, a second bar indicating that the user's glucose range is within the predefined target range for 54% of the predefined amount of time, and a third bar indicating that the user's glucose range is below the predefined target range for 12% of the predefined amount of time. Those of skill in the art will recognize that the percentages of time associated with each bar can vary depending on the available analyte data of the user. Furthermore, although FIG. 3E shows a predefined amount of time 332 equal to the last seven days and a predefined target range 334 of 80 to 140 mg/dL, those of skill in the art will appreciate that other predefined amounts of time (e.g., one day, three days, fourteen days, thirty days, ninety days, etc.) and/or predefined target ranges (e.g., 70 to 180 mg/dL) can be utilized, and are fully within the scope of the present disclosure.

FIG. 3F depicts another example embodiment of a Time-in-Ranges GUI 340, which includes a single bar comprising five bar portions including (from top to bottom): a first bar portion indicating that the user's glucose range is "Very High" or above 250 mg/dL for 1% (14 minutes) of a predefined amount of time, a second bar portion indicating that the user's glucose range is "High" or between 180 and 250 mg/dL for 18% (4 hours and 19 minutes) of the predefined amount of time, a third bar portion indicating that the user's glucose range is within a "Target Range" or between 70 and 180 mg/dL for 78% (18 hours and 43 minutes) of the predefined amount of time, a fourth bar portion indicating that the user's glucose range is "Low" or between 54 and 69 mg/dL for 3% (43 minutes) of the predefined amount of time, and a fifth bar portion indicating that the user's glucose range is "Very Low" or less than 54 mg/dL for 0% (0 minutes) of the predefined amount of time.

According to one aspect of the embodiment shown in FIG. 3F, each bar portion of Time-in-Ranges GUI 340 can comprise a different color. In some embodiments, bar portions can be separated by dashed or dotted lines 342 and/or interlineated with numeric markers 344 to indicate the ranges reflected by the adjacent bar portions. In some embodiments, the time in ranges reflected by the bar portions can be further expressed as a percentage, an actual amount of time (e.g., 4 hours and 19 minutes), or, as shown in FIG. 3F, both. Furthermore, those of skill in the art will recognize that the percentages of time associated with each bar portion can vary depending on the analyte data of the user. In some embodiments of Time-in-Ranges GUI 340, the Target Range can be configured by the user. In other embodiments, the Target Range of Time-in-Ranges GUI 340 is not modifiable by the user.

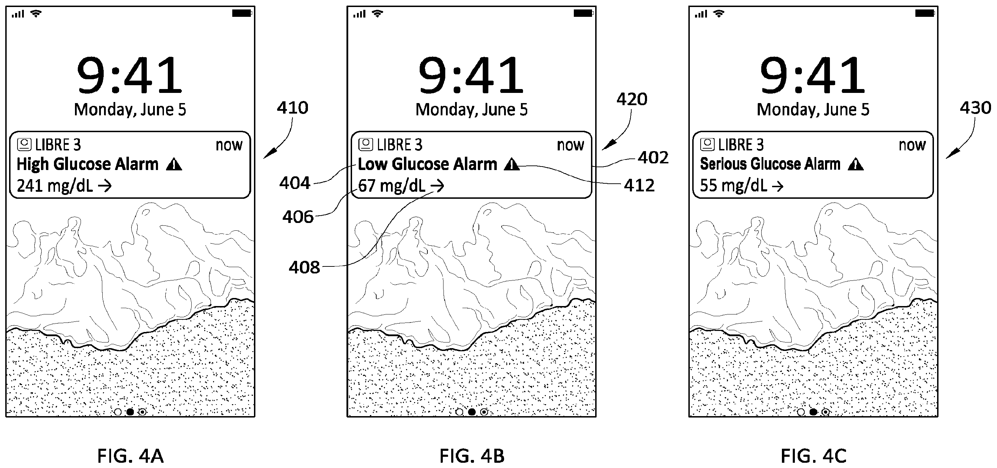

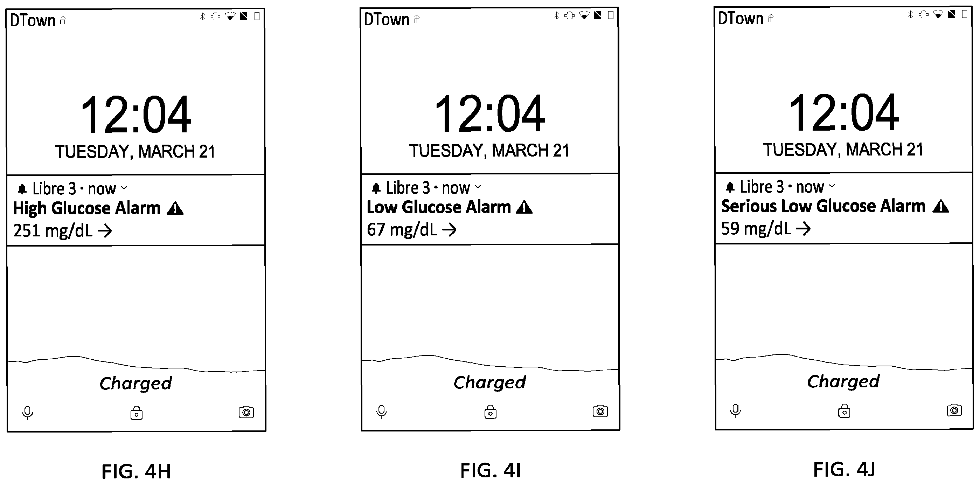

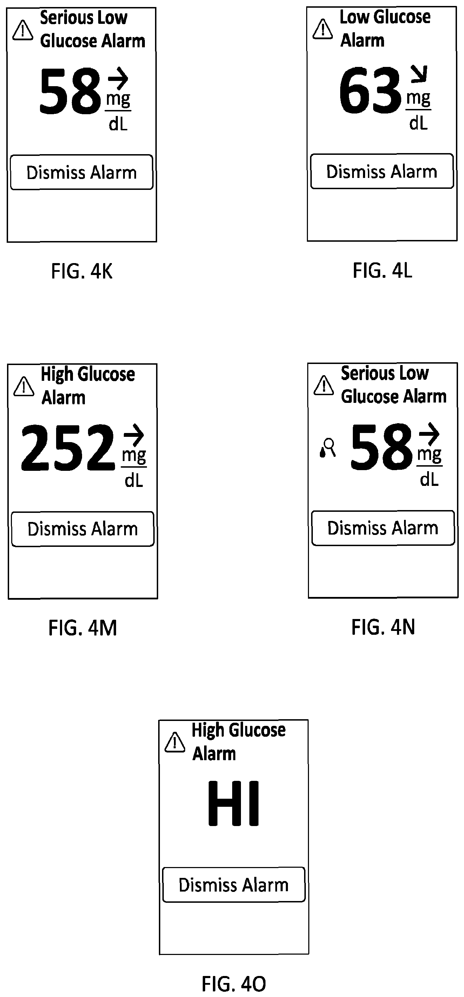

FIGS. 4A to 4O depict example embodiments of Analyte Level/Trend Alert GUIs for analyte monitoring systems. According to one aspect of the embodiments, the Analyte Level/Trend Alert GUIs comprise a visual notification (e.g., alert, alarm, pop-up window, banner notification, etc.), wherein the visual notification includes an alarm condition, an analyte level measurement associated with the alarm condition, and a trend indicator associated with the alarm condition.

Turning to FIGS. 4A to 4C, example embodiments of a High Glucose Alarm 410, Low Glucose Alarm 420, and a Serious Low Glucose Alarms 430 (sometimes, also referred to as "an Urgent Low Glucose Alarm") are depicted, respectively, wherein each alarm comprises a pop-up window 402 containing an alarm condition text 404 (e.g., "Low Glucose Alarm"), an analyte level measurement 406 (e.g., 67 mg/dL) associated with the alarm condition, and a trend indicator 408 (e.g., trend arrow) associated with the alarm condition. In some embodiments, an alarm icon 412 can be adjacent to the alarm condition text 404.

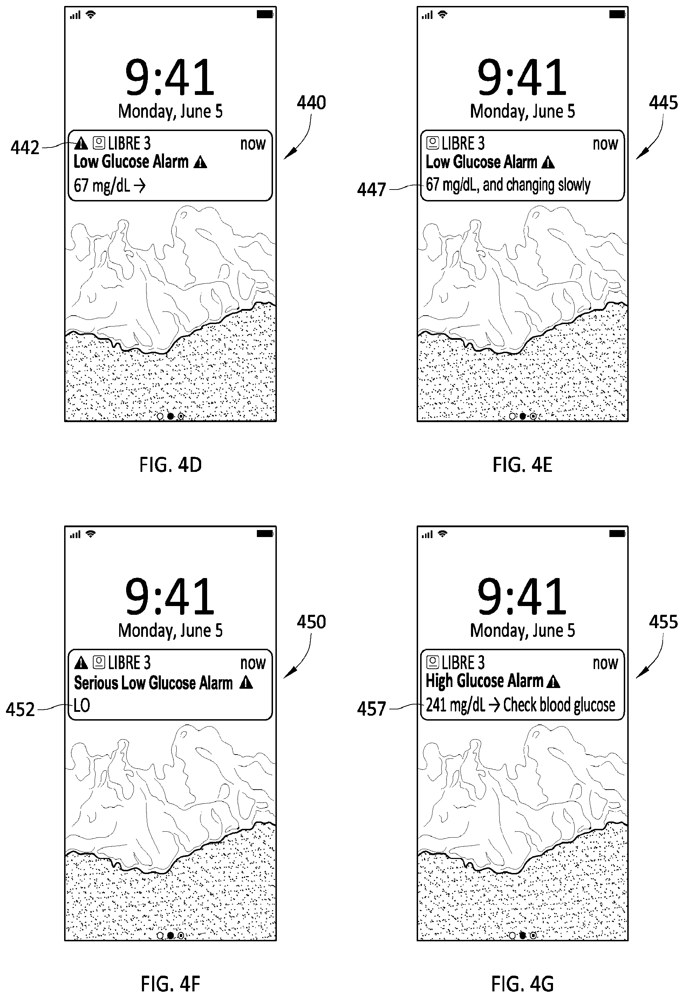

Referring next to FIGS. 4D to 4G, additional example embodiments of Low Glucose Alarms 440, 445, Serious Low Glucose Alarm 450, and High Glucose Alarm 455 are depicted, respectively. As shown in FIG. 4D, Low Glucose Alarm 440 is similar to the Low Glucose Alarm of FIG. 4B (e.g., comprises a pop-up window containing an alarm condition text, an analyte level measurement associated with the alarm condition, and a trend indicator associated with the alarm condition), but further includes a critical alert icon 442 to indicate that the alarm has been configured as a critical alert (e.g., will display, play a sound, vibrate, even if the device is locked or if the device's "Do Not Disturb" setting has been enabled). With respect to FIG. 4E, Low Glucose Alarm 445 is also similar to the Low Glucose Alarm of FIG. 4B, but instead of including a trend arrow, Log Glucose Alarm 445 includes a textual trend indicator 447. According to one aspect of some embodiments, textual trend indicator 447 can be enabled through a device's Accessibility settings such that the device will "read" the textual trend indicator 447 to the user via the device's text-to-speech feature (e.g., Voiceover for iOS or Select-to-Speak for Android).

Referring next to FIG. 4F, Low Glucose Alarm 450 is similar to the Low Glucose Alarm of FIG. 4D (including the critical alert icon), but instead of displaying an analyte level measurement associated with an alarm condition and a trend indicator associated with the alarm condition, Low Glucose Alarm 450 displays a out-of-range indicator 452 to indicate that the current glucose level is either above or below a predetermined reportable analyte level range (e.g., "HI" or "LO"). With respect to FIG. 4G, High Glucose Alarm 455 is similar to the High Glucose Alarm of FIG. 4A (e.g., comprises a pop-up window containing an alarm condition text, an analyte level measurement associated with the alarm condition, and a trend indicator associated with the alarm condition), but further includes an instruction to the user 457. In some embodiments, for example, the instruction can be a prompt for the user to "Check blood glucose." Those of skill in the art will appreciate that other instructions or prompts can be implemented (e.g., administer a corrective bolus, eat a meal, etc.).

Furthermore, although FIGS. 4A to 4G depict example embodiments of Analyte Level/Trend Alert GUIs that are displayed on smart phones having an iOS operating system, those of skill in the art will also appreciate that the Analyte Level/Trend Alert GUIs can be implemented on other devices including, e.g., smart phones with other operating systems, smart watches, wearables, reader devices, tablet computing devices, blood glucose meters, laptops, desktops, and workstations, to name a few. FIGS. 4H to 4J, for example, depict example embodiments of a High Glucose Alarm, Low Glucose Alarm, and a Serious Low Glucose Alarm for a smart phone having an Android Operating System. Similarly, FIGS. 4K to 4O depict, respectively, example embodiments of a Serious Low Glucose Alarm, Low Glucose Alarm, High Glucose Alarm, Serious Low Glucose Alarm (with a Check Blood Glucose icon), and High Glucose Alarm (with an out-of-range indicator) for a reader device. As described above, the Serious Low Glucose Alarm is sometimes also referred to as an Urgent Low Glucose Alarm, and can display the text "Urgent Low Glucose Alarm" in place of "Serious Low Glucose Alarm."

While the embodiments are susceptible to various modifications and alternative forms, specific examples thereof have been shown in the drawings and are herein described in detail.

## Claims

1. An analyte monitoring system (100), comprising:
an on-body unit comprising an analyte sensor and sensor electronics, wherein the on-body unit is configured to transmit data indicative of an analyte level of a user; and
a reader device (120) comprising a display (122), a transceiver configured to receive the data indicative of the analyte level, and a memory coupled with one or more processors, wherein the memory is configured to store instructions that, when executed by the one or more processors, cause the one or more processors to:
output to the display a first view comprising a first set of graphical elements, wherein, in the first view, each graphical element of the first set is representative of an amount of time that the analyte level of the user is within a predefined analyte range associated with a correlating graphical element of the first set, and wherein at least one analyte range associated with the first set is customizable by the user, and
**characterised in that** the instructions that, when executed by the one or more processors, further cause the one or more processors to:
output to the display a second view comprising a second set of graphical elements, wherein, in the second view, each graphical element of the second set is representative of an amount of time that the analyte level of the user is within a predefined analyte range associated with a correlating graphical element of the second set, and wherein none of the analyte ranges associated with the second set are customizable by the user, and
output to the display a switch element, wherein the switch element is configured to cause the first view or the second view to output to the display of the reader device.

2. The analyte monitoring system (100) of claim 1, wherein the switch element comprises a toggle, a switch, or a slidable element (310).

3. The analyte monitoring system (100) of claim 1, wherein the at least one analyte range associated with the first set that is customizable by the user comprises a customizable target analyte range.

4. The analyte monitoring system (100) of claim 1, wherein each of the graphical elements of the first set comprises a different color from the other graphical elements of the first set.

5. The analyte monitoring system (100) of claim 1, wherein each of the graphical elements of the second set comprises a different color from the other graphical elements of the second set.

6. The analyte monitoring system (100) of claim 1, wherein a total number of graphical elements of the first set is equal to a total number of graphical elements of the second set.

7. The analyte monitoring system (100) of claim 1, wherein a total number of graphical elements of the first set is not equal to a total number of graphical elements of the second set.

8. The analyte monitoring system (100) of claim 1, wherein the reader device (120) comprises a smart phone or a smart watch.

9. The analyte monitoring system (100) of claim 1, wherein the correlating amount of time that the analyte level of the user is within the analyte range associated with the each graphical element of the first set comprises a percentage of a first predefined time period, and wherein the correlating amount of time that the analyte level of the user is within the analyte range associated with the each graphical element of the second set comprises a percentage of a second predefined time period.

10. The analyte monitoring system (100) of claim 1, wherein the data indicative of the analyte level comprises data indicative of a glucose level in a bodily fluid of the user.

11. The analyte monitoring system (100) of claim 1, wherein the instructions, when executed by the one or more processors, further cause the one or more processors to output to the display (122) a date range indicator comprising a date range associated with the first set of graphical elements and the second set of graphical elements.

12. The analyte monitoring system (100) of claim 1, wherein the instructions, when executed by the one or more processors, further cause the one or more processors to output to the display (122) a data availability indicator comprising a period of time in which the data indicative of the analyte level is available.

13. The analyte monitoring system (100) of claim 1, wherein the instructions, when executed by the one or more processors, further cause the one or more processors to output to the display (122) a plurality of selectable icons configured to allow a user to select a predefined amount of time associated with the data indicative of the analyte level.

14. The analyte monitoring system (100) of claim 3, wherein the instructions, when executed by the one or more processors, further cause the one or more processors to output to the display (122) a link to an interface configured to allow the user to edit the customizable target analyte range.

## Patentansprüche

1. Ein Analytenüberwachungssystem (100), das Folgendes umfasst:
eine am Körper getragene Einheit, die einen Analytensensor und Sensorelektronik umfasst, wobei die am Körper getragene Einheit dazu ausgelegt ist, Daten zu übermitteln, die einen Analytenspiegel eines Benutzers anzeigen; und
ein Lesegerät (120), das eine Anzeige (122), einen Transceiver, der zum Empfangen der Daten, die den Analytenspiegel anzeigen, ausgelegt ist, und einen Speicher, der mit einem oder mehreren Prozessoren gekoppelt ist, umfasst, wobei der Speicher dazu ausgelegt ist, Anweisungen zu speichern, die, wenn sie von dem einen oder den mehreren Prozessoren ausgeführt werden, bewirken, dass der eine oder die mehreren Prozessoren Folgendes tun:
Ausgeben, zu der Anzeige, einer ersten Ansicht, die einen ersten Satz von Grafikelementen umfasst, wobei, in der ersten Ansicht, jedes Grafikelement des ersten Satzes eine Zeitdauer repräsentiert, über die der Analytenspiegel des Benutzers innerhalb eines vordefinierten Analytenbereichs liegt, der mit einem korrelierenden Grafikelement des ersten Satzes assoziiert ist, und wobei mindestens ein Analytenbereich, der mit dem ersten Satz assoziiert ist, durch den Benutzer anpassbar ist, und
**dadurch gekennzeichnet, dass** die Anweisungen, wenn sie von dem einen oder den mehreren Prozessoren ausgeführt werden, ferner bewirken, dass der eine oder die mehreren Prozessoren Folgendes tun:
Ausgeben, zu der Anzeige, einer zweiten Ansicht, die einen zweiten Satz von Grafikelementen umfasst, wobei, in der zweiten Ansicht, jedes Grafikelement des zweiten Satzes eine Zeitdauer repräsentiert, über die der Analytenspiegel des Benutzers innerhalb eines vordefinierten Analytenbereichs liegt, der mit einem korrelierenden Grafikelement des zweiten Satzes assoziiert ist, und wobei keiner der Analytenbereiche, die mit dem zweiten Satz assoziiert sind, durch den Benutzer anpassbar ist, und
Ausgeben, zu der Anzeige, eines Wechselelements, wobei das Wechselelement dazu ausgelegt ist, das Ausgeben der ersten Ansicht oder der zweiten Ansicht zu der Anzeige des Lesegeräts zu bewirken.

2. Analytenüberwachungssystem (100) nach Anspruch 1, wobei das Wechselelement einen Kippschalter, einen Schalter oder ein verschiebbares Element (310) umfasst.

3. Analytenüberwachungssystem (100) nach Anspruch 1, wobei der mindestens eine Analytenbereich, der mit dem ersten Satz assoziiert ist, der durch den Benutzer anpassbar ist, einen anpassbaren Zielanalytenbereich umfasst.

4. Analytenüberwachungssystem (100) nach Anspruch 1, wobei jedes der Grafikelemente des ersten Satzes eine von den anderen Grafikelementen des ersten Satzes verschiedene Farbe umfasst.

5. Analytenüberwachungssystem (100) nach Anspruch 1, wobei jedes der Grafikelemente des zweiten Satzes eine von den anderen Grafikelementen des zweiten Satzes verschiedene Farbe umfasst.

6. Analytenüberwachungssystem (100) nach Anspruch 1, wobei eine Gesamtzahl von Grafikelementen des ersten Satzes gleich einer Gesamtzahl von Grafikelementen des zweiten Satzes ist.

7. Analytenüberwachungssystem (100) nach Anspruch 1, wobei eine Gesamtzahl von Grafikelementen des ersten Satzes nicht gleich einer Gesamtzahl von Grafikelementen des zweiten Satzes ist.

8. Analytenüberwachungssystem (100) nach Anspruch 1, wobei das Lesegerät (120) ein Smartphone oder eine Smartwatch umfasst.

9. Analytenüberwachungssystem (100) nach Anspruch 1, wobei die korrelierende Zeitdauer, über die der Analytenspiegel des Benutzers innerhalb des Analytenbereichs liegt, der mit jedem Grafikelement des ersten Satzes assoziiert ist, einen Prozentanteil eines ersten vordefinierten Zeitraums umfasst, und wobei die korrelierende Zeitdauer, über die der Analytenspiegel des Benutzers innerhalb des Analytenbereichs liegt, der mit jedem Grafikelement des zweiten Satzes assoziiert ist, einen Prozentanteil des zweiten vordefinierten Zeitraums umfasst.

10. Analytenüberwachungssystem (100) nach Anspruch 1, wobei die Daten, die den Analytenspiegel anzeigen, Daten umfassen, die einen Glukosespiegel in einer Körperflüssigkeit des Benutzers anzeigen.

11. Analytenüberwachungssystem (100) nach Anspruch 1, wobei die Anweisungen, wenn sie von dem einen oder den mehreren Prozessoren ausgeführt werden, ferner bewirken, dass der eine oder die mehreren Prozessoren einen Datenbereichsindikator zu der Anzeige (122) ausgeben, der einen Datenbereich umfasst, der mit dem ersten Satz von Grafikelementen und dem zweiten Satz von Grafikelementen assoziiert ist.

12. Analytenüberwachungssystem (100) nach Anspruch 1, wobei die Anweisungen, wenn sie von dem einen oder den mehreren Prozessoren ausgeführt werden, ferner bewirken, dass der eine oder die mehreren Prozessoren einen Datenverfügbarkeitsindikator zu der Anzeige (122) ausgeben, der einen Zeitraum umfasst, in dem die Daten, die den Analytenspiegel anzeigen, verfügbar sind.

13. Analytenüberwachungssystem (100) nach Anspruch 1, wobei die Anweisungen, wenn sie von dem einen oder den mehreren Prozessoren ausgeführt werden, ferner bewirken, dass der eine oder die mehreren Prozessoren eine Vielzahl von auswählbaren Symbolen zu der Anzeige (122) ausgeben, die dazu ausgelegt sind, einem Benutzer die Auswahl einer vordefinierten Zeitdauer zu erlauben, die mit den Daten assoziiert ist, die den Analytenspiegel anzeigen.

14. Analytenüberwachungssystem (100) nach Anspruch 3, wobei die Anweisungen, wenn sie von dem einen oder den mehreren Prozessoren ausgeführt werden, ferner bewirken, dass der eine oder die mehreren Prozessoren einen Link zu einer Schnittstelle zu der Anzeige (122) ausgeben, die dazu ausgelegt ist, dem Benutzer das Editieren des anpassbaren Zielanalytenbereichs zu erlauben.

## Revendications

1. Système de surveillance d'analyte (100), comprenant :
une unité sur le corps comprenant un capteur d'analyte et un élément électronique de capteur, dans lequel l'unité sur le corps est configurée pour transmettre des données indiquant un niveau d'analyte d'un utilisateur ; et
un dispositif de lecteur (120) comprenant un dispositif d'affichage (122), un émetteur-récepteur configuré pour recevoir les données indiquant le niveau d'analyte, et une mémoire couplée à un ou plusieurs processeurs, dans lequel la mémoire est configurée pour stocker des instructions qui, lorsqu'elles sont exécutées par les un ou plusieurs processeurs, amènent les uns ou plusieurs processeurs à :
sortir sur le dispositif d'affichage une première vue comprenant un premier ensemble d'éléments graphiques, dans lequel, dans la première vue, chaque élément graphique du premier ensemble est représentatif d'une quantité de temps pendant laquelle le niveau d'analyte de l'utilisateur est compris dans les limites d'une plage d'analyte prédéfinie associée à un élément graphique de corrélation du premier ensemble, et dans lequel au moins une plage d'analyte associée au premier ensemble est personnalisable par l'utilisateur, et
**caractérisé en ce que** les instructions qui, lorsqu'elles sont exécutées par les un ou plusieurs processeurs, amènent en outre les un ou plusieurs processeurs à :
sortir sur le dispositif d'affichage une deuxième vue comprenant un deuxième ensemble d'éléments graphiques, dans lequel, dans la deuxième vue, chaque élément graphique du deuxième ensemble est représentatif d'une quantité de temps pendant laquelle le niveau d'analyte de l'utilisateur est compris dans les limites d'une plage d'analyte prédéfinie associée à un élément graphique de corrélation du deuxième ensemble, et dans lequel aucune des plages d'analyte associées au deuxième ensemble n'est personnalisable par l'utilisateur, et
sortir sur le dispositif d'affichage un élément de commutation, dans lequel l'élément de commutation est configuré pour amener la première vue ou la deuxième vue à sortir sur le dispositif d'affichage du dispositif de lecteur.

2. Système de surveillance d'analyte (100) selon la revendication 1, dans lequel l'élément de commutation comprend un interrupteur, un commutateur ou un élément capable de coulisser (310).

3. Système de surveillance d'analyte (100) selon la revendication 1, dans lequel l'au moins une plage d'analyte associée au premier ensemble qui est personnalisable par l'utilisateur comprend une plage d'analyte cible personnalisable.

4. Système de surveillance d'analyte (100) selon la revendication 1, dans lequel chacun des éléments graphiques du premier ensemble comprend une couleur différente des autres éléments graphiques du premier ensemble.

5. Système de surveillance d'analytes (100) selon la revendication 1, dans lequel chacun des éléments graphiques du deuxième ensemble comprend une couleur différente des autres éléments graphiques du deuxième ensemble.

6. Système de surveillance d'analytes (100) selon la revendication 1, dans lequel un nombre total d'éléments graphiques du premier ensemble est égal à un nombre total d'éléments graphiques du deuxième ensemble.

7. Système de surveillance d'analyte (100) selon la revendication 1, dans lequel un nombre total d'éléments graphiques du premier ensemble n'est pas égal à un nombre total d'éléments graphiques du deuxième ensemble.

8. Système de surveillance d'analyte (100) selon la revendication 1, dans lequel le dispositif de lecteur (120) comprend un téléphone intelligent ou une montre intelligente.

9. Système de surveillance d'analyte (100) selon la revendication 1, dans lequel la quantité de temps de corrélation pendant laquelle le niveau d'analyte de l'utilisateur est compris dans les limites de la plage d'analyte associée à chaque élément graphique du premier ensemble comprend un pourcentage d'une première période de temps prédéfinie, et dans lequel la quantité de temps de corrélation pendant laquelle le niveau d'analyte de l'utilisateur est compris dans les limites de la plage d'analyte associée à chaque élément graphique du deuxième ensemble comprend un pourcentage d'une deuxième période de temps prédéfinie.

10. Système de surveillance d'analyte (100) selon la revendication 1, dans lequel les données indiquant le niveau d'analyte comprennent des données indiquant un niveau de glucose dans un fluide corporel de l'utilisateur.

11. Système de surveillance d'analytes (100) selon la revendication 1, dans lequel les instructions, lorsqu'elles sont exécutées par les un ou plusieurs processeurs, amènent en outre les un ou plusieurs processeurs à sortir sur le dispositif d'affichage (122) un indicateur de plage de dates comprenant une plage de dates associée au premier ensemble d'éléments graphiques et au deuxième ensemble d'éléments graphiques.

12. Système de surveillance d'analyte (100) selon la revendication 1, dans lequel les instructions, lorsqu'elles sont exécutées par les un ou plusieurs processeurs, amènent en outre les un ou plusieurs processeurs à sortir sur le dispositif d'affichage (122) un indicateur de disponibilité de données comprenant une période de temps pendant laquelle les données indiquant le niveau d'analyte sont disponibles.

13. Système de surveillance d'analyte (100) selon la revendication 1, dans lequel les instructions, lorsqu'elles sont exécutées par les un ou plusieurs processeurs, amènent en outre les un ou plusieurs processeurs à sortir sur le dispositif d'affichage (122) une pluralité d'icônes sélectionnables configurées pour permettre à un utilisateur de sélectionner une quantité de temps prédéfinie associée aux données indiquant le niveau d'analyte.

14. Système de surveillance d'analytes (100) selon la revendication 3, dans lequel les instructions, lorsqu'elles sont exécutées par les un ou plusieurs processeurs, amènent en outre les un ou plusieurs processeurs à sortir sur le dispositif d'affichage (122) un lien vers une interface configurée pour permettre à l'utilisateur d'éditer la plage d'analyte cible personnalisable.
